# EUROPEAN PATENT SPECIFICATION

(11) **EP 0 348 052 B1**
(45) Date of publication and mention of the grant of the patent: **09.04.1997**
(21) Application number: 89305536.8
(22) Date of filing: 01.06.1989
(51) Int. Cl.: A62B 17/00, A62B 17/04, B32B 27/08

(54) **Fire and smoke protective hood**
Feuer- und Rauchschutzhaube
Cagoule protectrice contre le feu et la fumée

(30) Priority: 22.06.1988 GB 8814786
(43) Date of publication of application: 27.12.1989
(73) Proprietor: McKenzie, Noel Robertson, Worthing, West Sussex BN11 4DX (GB)
(72) Inventor: McKenzie, Noel Robertson, Worthing, West Sussex BN11 4DX (GB)
(74) Representative: Lamb, John Baxter

(56) References cited:
- EP-A- 0 049 083
- EP-A- 0 178 856
- EP-A- 0 197 641
- DE-A- 2 904 100
- GB-A- 1 372 694
- GB-A- 2 031 498
- PATENT ABSTRACTS OF JAPAN vol. 015, no. 215 (M-1119)31 May 1991 & JP-A-30 061 037 (TOMIZO YAMAMOTO) 15 March 1991

## Description

This invention relates to protective hoods and in particular to a protective hood for protecting an individual from the effects of smoke and fire in a fire related emergency.

It is known to provide a protective hood in the form of a bag of heat resistant plastics material which may be used in the event of a fire related emergency to protect an individual from the effects of smoke and fire. Such protective hoods may be pulled over the head of the individual and provide a limited volume of clean, smoke-free air which may suffice to sustain the individual whilst they attempt to escape from the fire related emergency. Such hoods may suitably be used in fire related emergencies in confined spaces, such as hotels, factories, homes, vehicles, ships and aircraft. The limited amount of clean air may be provided by means of suitable filters or by compressed oxygen or air supplies to the hood.

However, such hoods tend to transmit and absorb heat from the fire so that the individual's head is not protected from the effects of heat, for example discomfort, burns and the like.

It is known to provide such hoods with a metal coating to reflect heat and reduce absorption. Such coatings may comprise a layer of gold, silver and aluminium. However, it has been found that some coatings tend to be dislodged from the heat resistant plastics material under the conditions to which they are exposed in a fire related emergency. Furthermore, some metals tend to be attacked by the noxious gases to which they are exposed in a fire related emergency and may become opaque.

Thus according to the present invention there is provided a protective hood for protecting an individual from the effects of smoke and fire in a fire related emergency, comprising a high temperature resistant plastics material having a layer of titanium on at least a part of its outer surface.

Preferably, the plastics material is a thermoset plastics material such as polyimide, for example Upilex (trademark) or Kapton (trademark). Preferably, at least a part of the plastics material is transparent to visible radiation.

Preferably, the hood has a layer of fluoropolymer on at least a part of its inner surface. The fluoropolymer may be fluoroethylene polymer (FEP) or perfluoroalkoxy polymer (PFA).

The fluoropolymer may be in the form of a layer 10 to 40 micrometres thick. The plastics material may be in the form of a film 25 to 75 micrometres thick.

The layer of titanium is preferably sufficiently thick to provide the required reflection and transmission properties for heat, but if the hood is used to cover the eyes of the individual, the layer of titanium which covers the eyes must still be sufficiently transparent to visible radiation to provide sufficient visibility for the individual. This may be achieved by using a layer of titanium of different thicknesses at different parts of the hood. The titanium may be a layer several hundred angstroms thick, that is between 100 and 1000 angstroms (10-100 nm) thick. The titanium may be between 50 and 200 mg per square metre of hood material, preferably between 100 and 150 mg per square metre. The titanium may be applied by sputtering. The hood material may have a transmittance of electromagnetic radiation of between 10% and 40%, preferably between 15 and 25% at 620 nanometres. Preferably, the hood material has about a 70% rejection of infra red radiation.

The present invention may also be used in the form of a cloak or other garment which may be placed over part or all of the body of the individual.

The present invention may be used in the form of a visor on a protective mask or in the form of shield which may be used in a fire related emergency.

Also according to the present invention there is provided an emergency breathing apparatus comprising a hood as hereinbefore described and having suitable filters or a breathable gas supply to provide a wearer of the hood with a limited volume of clean, smoke-free breathable gas which may suffice to sustain the individual whilst they attempt to escape from the fire related emergency. The breathable gas supply may be an independent compressed oxygen-containing gas supply.

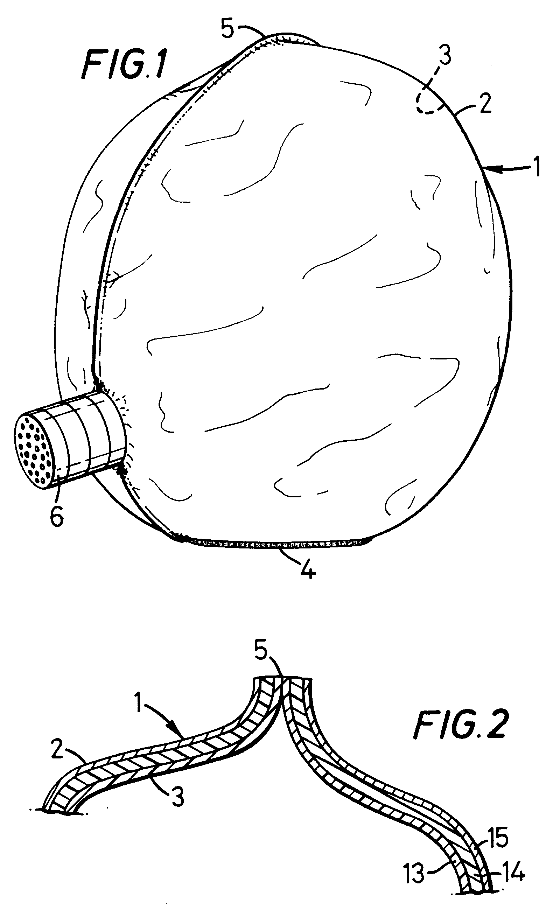

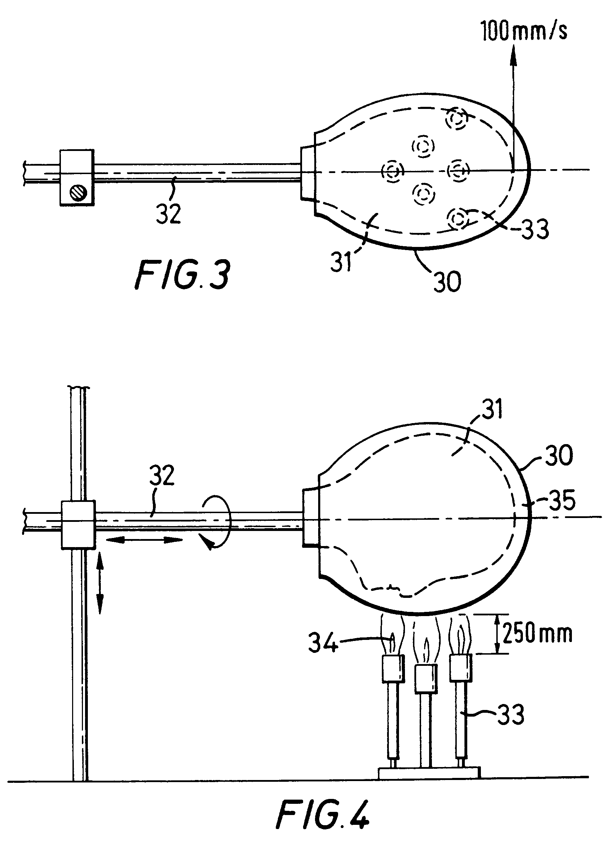

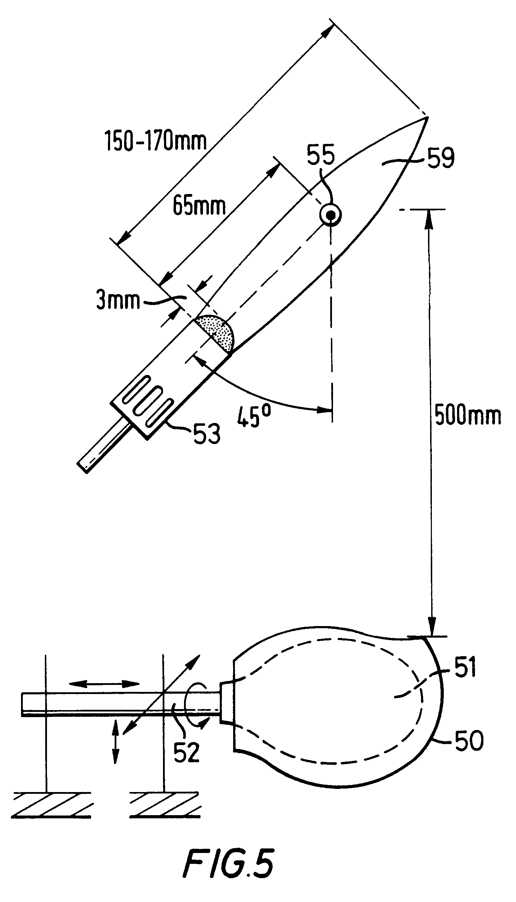

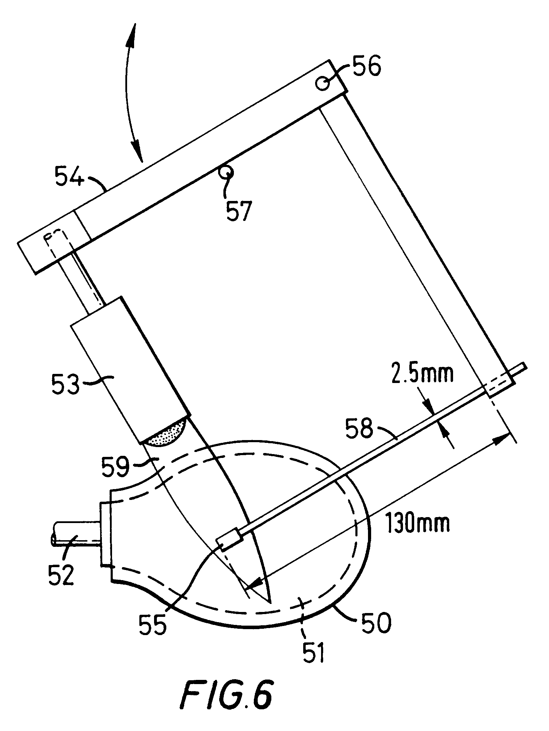

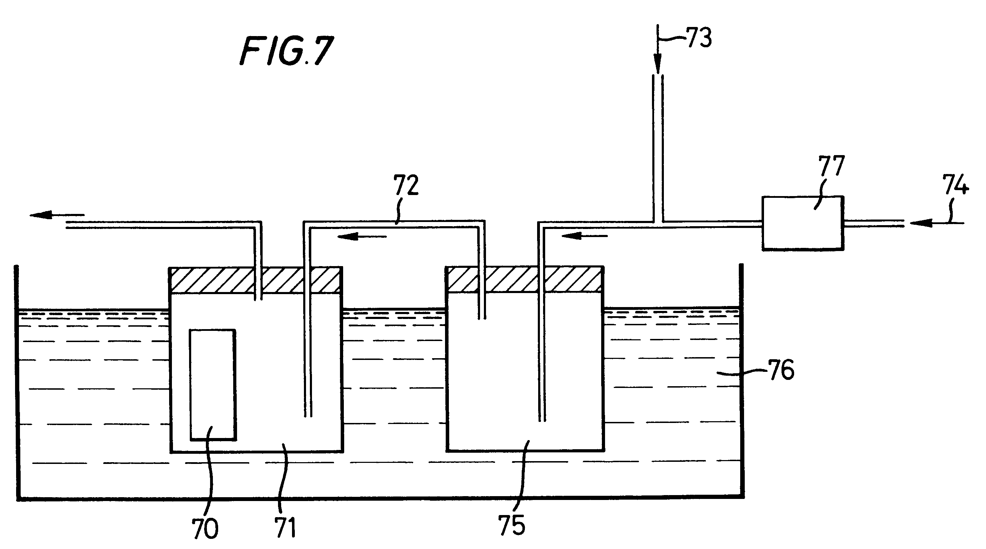

The invention will now be described by way of example only and with reference to the accompanying drawings. Figure 1 represents a protective hood according to the present invention and Figure 2 represents, in cross-section, part of a protective hood according to the present invention. Figure 3 is a plan view of the apparatus used for testing the flame resistance of a protective hood according to the present invention. Figure 4 is a side elevation of the apparatus shown in Figure 3. Figure 5 is a side elevation view of the apparatus used for testing the resistance to molten drops of nylon of a protective hood according to the present invention. Figure 6 is a plan view of the apparatus shown in Figure 5. Figure 7 is a schematic view of the apparatus used to test the chemical resistance of material used to fabricate protective hoods according to the present invention.

In Figure 1, a protective hood (1) according to the present invention comprises Kapton having a layer of titanium on its outer surface (2) and a layer of fluoropolymer on its inner surface (3). The hood has a suitable neck seal (4) which allows the hood to be pulled over the head of an individual (not shown) in a fire related emergency and forms a seal with the neck (not shown) to prevent the ingress of smoke and fumes. The hood is fabricated by joining suitably shaped pieces of Kapton film at suitable seams (5) by heat and pressure welding. The hood is provided with filters (6) to allow the individual to breath clean, smoke-free air for a limited period whilst wearing the hood.

In Figure 2 part of a hood (1) according to the present invention is shown in cross-section with an inner surface (3) adjacent to the head of an individual (not shown) and an outer surface (2) adjacent to a source of heat or fire (not shown). The hood (1) comprises three layers, an inner layer of fluoroethylene polymer (13), 25 micrometres thick, a layer of Kapton film (14), 50 micrometres thick and an outer layer of sputtered titanium (15) several hundred angstroms thick. Figure 2 also shows a seam (5) of two pieces of the Kapton joined to form the hood so that the inner (fluoroethylene polymer) layers (13) are bonded together to provide a gas-tight seal.

Prototype hoods without filters fabricated from material comprising fluoroethylene polymer, 25 micrometres thick; Kapton, 50 micrometres thick and titanium were subjected to a flame exposure test. The titanium was sputtered onto the Kapton by a DC magnetron sputtering process with argon partial pressure. The amount of titanium on the Kapton was measured by standard wet ashing analysis to be 116 milligrams of titanium per square metre which by calculation, is believed to be equivalent to a thickness of about 255 angstroms. Figures 3 and 4 show the apparatus used for the flame exposure test. A hood (30) according to the example was supported on a metal head-shaped holder (31) called a Sheffield head. The hood (30) contained air (35). The Sheffield head (31) was held on a support (32) which was adjustable. Six burners (33) were used to produce a large diffuse propane flame (34) using a propane supply of 13 litres (NTP) per minute at 0.25 barg. These burners (33) produced a flame (34) with a temperature maximum of 915°C to 920°C. The smoke hood (30) was positioned 250 mm above the burners. The burners (33) had adjustable height for this purpose. The hood (30) was passed through the flame (34) from the burners (33) at a traverse rate of 100 mm/s giving a flame contact time of 5 to 6 seconds. The heat flux in the flame was about 40 Kw/m². The main areas of the hoods and the seams were exposed in separate tests by changing the position of the hood (30) on the Sheffield head (31). A limited number of more severe tests were undertaken by passing the hood more than once through the flame and by using a larger flame.

The titanium coated Kapton FEP of the hood showed no significant effect of the flame (34) after a number (up to three) passes through the flame. Some particulate matter (soot) was deposited on the surface but was easily wiped off. There was no obvious attenuation in the transparency of the hood material, in fact the material appeared more transparent after the flame tests.

The seams of the hood withstood contact with the flame (34) when the hood (30) was passed once through the flame in both horizontal and vertical orientations, i.e. seam facing down towards the flame (34). In view of this lack of damage, a limited number of more severe tests were carried out in which a hood was passed repeatedly through the flame, the hood resisted a single pass and also a second pass, 2 minutes after the first, and the seam only started to fail after a third pass. A limited number of tests were also undertaken with a larger propane flame (propane supply at 1.25 bar and 27 litres (NTP)/minute), the hood resisted two passes through the larger flame and the seam only started to fail in areas of high stress (hoods were a tight fit on the Sheffield head) during the third pass.

Prototype hoods without filters fabricated from material comprising FEP, 25 micrometres thick; Kapton, 50 micrometres thick and titanium, 116 milligrams per square metre were also subjected to molten drop tests. Figures 5 and 6 show the apparatus used for this test. A hood (50) according to the example was supported on a rubber head-shaped holder (51) called a Sheffield head. The Sheffield head (51) was held on a support (52) which was adjustable. A gas burner (53) on a swivel mounting (54) was mounted above the hood (50). The gas burner produced a flame (59) 150-170mm long with a temperature of about 1050°C using commercial grade propane gas at 1 barg 1.2 litres (NTP) per minute. A piece of nylon 11 tubing (55) was held on a support (58) 500mm above the hood (50). The swivel mounting (54) was pivotable about a pivot (56) and a stop (57) prevented the burner from being moved closer than 65mm to the nylon tubing (55). The nylon 11 tube (55) had a length of 10mm, an outside diameter of 2.5 mm, internal diameter of 1.7 mm, a melting point of 170°C and contained 11% butylbenzene sulphonamide plasticiser. It also had a Melt Flow Index 230°C 2.16Kg 11g/10min Melt Flow Index 190°C 2.16Kg 1.8g/10 min.

During the test, the burner (53) produced a flame (59) which melted the nylon tube (55) and caused burning drops of nylon to fall onto the hood (51). The drops typically burned for about 4 to 8 seconds. Both the main area of the hood and the seams were tested separately. The tests showed that the drops burned for several seconds before extinuishing without causing any damage to the hood material. The hoods were not significantly distorted or penetrated by the molten drops. When cool the drops were easily removed from the hood leaving an undamaged surface.

Samples of titanium coated plastics materials were evaluated for resistance to various noxious gases which might be expected to be present in the atmosphere of a fire related emergency. For comparison, samples of stainless steel coated polyester were also assessed. The effects of the various chemicals were assessed visually and by the effect on optical properties (% transmittance of different incident electromagnetic radiation wavelengths using a Perkin Elmer Lambda 9 UV/VIS/NIR Spectrometer). The apparatus used for exposing the samples to the noxious gas is shown schematically in Figure 7. The samples (70) of material were placed in a polypropylene exposure vessel (71) through which a stream of gas (72) was passed. The gas (72) comprised a mixture of concentrated noxious gas (73) and air (74) which were premixed and preheated in a preheating vessel (75). Both the preheating vessel (75) and the exposure vessel (71) were kept at a constant temperature (100°C) in a thermostatically controlled water bath (76).

The samples (70) were exposed for 30 minutes at 100°C to humid and dry test atmospheres separately. Humid conditions were obtained by passing the air through a water-filled Drechsel bottle (77) fitted with a mist trap and corresponded to approximately 90% relative humidity. Dry conditions were obtained by replacing the Drechsel bottle with a drying tower and passing the air through the drying tower which contained, for example, phosphorous pentoxide and corresponded to less than 5% relative humidity.

The samples (70) were exposed to test atmospheres on one face only (the metal coated side where applicable) the rear face being protected from exposure by taping the samples onto a sheet of PTFE (not shown).

The following noxious gases were used separately, all at 1000 vapour parts per million: hydrogen chloride, hydrogen cyanide, hydrogen fluoride, ammonia, nitrogen dioxide, sulphur dioxide. The samples were also exposed to all these gases sequentially in the order given in the tables. The results are tabulated in Tables 1 to 11.

Tables 1 and 2 show comparative results for stainless steel coated polyester. Tables 3 and 4 show results for titanium coated polyester. The stainless steel and titanium were sputtered onto the polyester by a DC magnetron sputtering process with argon partial pressure. The polyester was 142 gauge. Table 5 shows the results for the same material as was used for the flame tests, that is FEP 25 micrometres thick, Kapton 50 micrometres thick and 116 milligrams of titanium per square metre. This sample had a transmittance of 19% at 620mm and a similar sample had a transmittance of 21% of 620mm, measured using the Lambda 9 spectrometer. Tables 6 to 11 inclusive show results for various titanium coated Kapton/FEP samples with ammonia and hydrogen fluoride. The thickness of the titanium for the samples in Tables 6 to 11 was measured on-line by an optical monitor within the sputtering machine at 620 nanometre wavelength and is referred to as %T which is the percentage of energy transmitted at 620 nanometres.

The stainless steel coated materials were affected only by hydrogen fluoride and hydrogen chloride. The film damage was only just discernable visually but the transmittance properties in Tables 1 and 2 show an increase in transmittance indicative of attack of the stainless steel.

Titanium coated polyester showed no damage from any of the gases except hydrogen fluoride. Some very slight visual damage was discernable with hydrogen fluoride and the results in Tables 3 and 4 show an increase in transmittance after exposure to hydrogen fluoride, indicative of attack. Whilst these results show the chemical resistance of titanium, polyester would not be a suitable plastics material according to the present invention.

Titanium coated Kapton/FEP showed no visible sign of attack by any of the gases but the transmittance properties shown in Table 5 show that there was slight attack by hydrogen fluoride, resulting in a very small increase in transmittance.

Similar results are shown in Tables 6 to 11 which show that hydrogen fluoride caused slight metallic layer damage to the titanium coated Kapton/FEP but ammonia did not.

The examples given show that the protective hood according to the present invention exhibits good resistance to the conditions which may be present in a fire related emergency, that is, good flame resistance, good resistance to molten, burning plastics material and good resistance to noxious gases.

**TABLE 1**

| EXPOSURE OF STAINLESS STEEL COATED POLYESTER TO NOXIOUS GASES | | | |
|---|---|---|---|
| Gas | % Transmittance of electromagnetic radiation at wavelength | | |
| | 600 nm | 1200 nm | 2000 nm |
| None | 47 | 51 | 51 |
| Hydrogen chloride (humid) | 51 | 57 | 57 |
| Hydrogen fluoride (humid) | 47 | 50 | 54 |
| Sulphur dioxide (humid) | 47 | 51 | 51 |
| Nitrogen dioxide (humid) | 47 | 53 | 54 |
| Hydrogen cyanide (humid) | 48 | 50 | 53 |
| All gases sequentially (humid) | 57 | 60 | 60 |

**TABLE 2**

| EXPOSURE OF STAINLESS STEEL COATED POLYESTER TO NOXIOUS GASES | | | |
|---|---|---|---|
| Gas | % Transmittance of electomagnetic radiation at wavelength | | |
| | 600 nm | 1200 nm | 2000 nm |
| None | 29 | 31 | 32 |
| Hydrogen chloride (humid) | 35 | 35 | 36 |
| Hydrogen fluoride (humid) | 35 | 35 | 35 |
| Sulphur dioxide (humid) | 30 | 30 | 33 |
| Nitrogen dioxide (humid) | 31 | 32 | 35 |
| Hydrogen cyanide (humid) | 31 | 30 | 36 |
| All gases sequentially (humid) | 37 | 38 | 37 |

**TABLE 3**

| EXPOSURE OF TITANIUM COATED POLYESTER TO NOXIOUS GASES | | | |
|---|---|---|---|
| Gas | % Transmittance of electromagnetic radiation at wavelength | | |
| | 600 nm | 1200 nm | 2000 nm |
| None | 47 | 49 | 49 |
| Hydrogen chloride (humid) | 49 | 47 | 48 |
| Hydrogen fluoride (humid) | 59 | 58 | 59 |
| Sulphur dioxide (humid) | 49 | 47 | 49 |
| Nitrogen dioxide (humid) | 50 | 47 | 50 |
| Hydrogen cyanide (humid) | 50 | 47 | 48 |
| All gases sequentially (humid) | 64 | 61 | 63 |

**TABLE 4**

| EXPOSURE OF TITANIUM COATED POLYESTER TO NOXIOUS GASES | | | |
|---|---|---|---|
| Gas | % Transmittance of electromagnetic radiation at wavelength | | |
| | 600 nm | 1200 nm | 2000 nm |
| None | 30 | 30 | 32 |
| Hydrogen chloride (humid) | 30 | 29 | 32 |
| Hydrogen fluoride (humid) | 35 | 35 | 37 |
| Sulphur dioxide (humid) | 30 | 30 | 30 |
| Nitrogen dioxide (humid) | 31 | 30 | 31 |
| Hydrogen cyanide (humid) | 30 | 29 | 29 |
| All gases sequentially (humid) | 39 | 36 | 37 |

**TABLE 5**

| EXPOSURE OF TITANIUM COATED KAPTON/FEP TO NOXIOUS GASES | | | |
|---|---|---|---|
| Gas | % Transmittance of electromagnetic radiation at wavelength | | |
| | 600 nm | 1200 nm | 2000 nm |
| None | 19 | 23 | 23 |
| Hydrogen chloride (humid) | 19 | 22 | 23 |
| Hydrogen chloride (dry) | 19 | 22 | 25 |
| Hydrogen fluoride (humid) | 22 | 26 | 28 |
| Hydrogen fluoride (dry) | 20 | 23 | 26 |
| Sulphur dioxide (humid) | 19 | 22 | 25 |
| Sulphur dioxide (dry) | 19 | 23 | 24 |
| Nitrogen dioxide (humid) | 20 | 24 | 26 |
| Nitrogen dioxide (dry) | 20 | 24 | 25 |
| Hydrogen cyanide (humid) | 19 | 23 | 24 |
| Hydrogen cyanide (dry) | 19 | 22 | 23 |
| Ammonia (humid) | 19 | 22 | 23 |
| Ammonia (dry) | 19 | 23 | 25 |
| All gases sequentially (humid) | 21 | 25 | 25 |

**TABLE 6**

| EXPOSURE OF TITANIUM COATED KAPTON/FEP (43.6%T) TO NOXIOUS GASES | | | |
|---|---|---|---|
| Gas | % Transmittance of electromagnetic radiation at wavelength | | |
| | 600 nm | 1200 nm | 2000 nm |
| None | 29 | 28 | 34 |
| Hydrogen fluoride (humid) | 30 | 29 | 34 |
| Ammonia (humid) | 22 | 24 | 28 |
| Sequentially exposed to both gases (humid) | 28 | 26 | 29 |

**TABLE 7**

| EXPOSURE OF TITANIUM COATED KAPTON/FEP (47.8%T) TO NOXIOUS GASES | | | |
|---|---|---|---|
| Gas | % Transmittance of electromagnetic radiation at wavelength | | |
| | 600 nm | 1200 nm | 2000 nm |
| None | 36 | 35 | 41 |
| Hydrogen fluoride (humid) | 38 | 34 | 38 |
| Ammonia (humid) | 37 | 35 | 39 |
| Sequentially exposed to both gases (humid) | 38 | 35 | 40 |

**TABLE 8**

| EXPOSURE OF TITANIUM COATED KAPTON/FEP (70.7%T) TO NOXIOUS GASES | | | |
|---|---|---|---|
| Gas | % Transmittance of electromagnetic radiation at wavelength | | |
| | 600 nm | 1200 nm | 2000 nm |
| None | 50 | 52 | 60 |
| Hydrogen fluoride (humid) | 58 | 64 | 70 |
| Ammonia (humid) | 49 | 51 | 58 |
| Sequentially exposed to both gases (humid) | 57 | 62 | 68 |

**TABLE 9**

| EXPOSURE OF TITANIUM COATED KAPTON/FEP (34.7%T) TO NOXIOUS GASES | | | |
|---|---|---|---|
| Gas | % Transmittance of electromagnetic radiation at wavelength | | |
| | 600 nm | 1200 nm | 2000 nm |
| None | 19 | 20 | 22 |
| Hydrogen fluoride (humid) | 25 | 25 | 26 |
| Ammonia (humid) | 22 | 22 | 26 |
| Sequentially exposed to both gases (humid) | 32 | 32 | 35 |

**TABLE 10**

| EXPOSURE OF TITANIUM COATED KAPTON/FEP (15.1%T) TO NOXIOUS GASES | | | |
|---|---|---|---|
| Gas | % Transmittance of electromagnetic radiation at wavelength | | |
| | 600 nm | 1200 nm | 2000 nm |
| None | 9 | 10 | 13 |
| Hydrogen fluoride (humid) | 14 | 14 | 16 |
| Ammonia (humid) | 8 | 9 | 12 |
| Sequentially exposed to both gases (humid) | 14 | 13 | 16 |

**TABLE 11**

| EXPOSURE OF TITANIUM COATED KAPTON/FEP (13.5%T) TO NOXIOUS GASES | | | |
|---|---|---|---|
| Gas | % Transmittance of electromagnetic radiation at wavelength | | |
| | 600 nm | 1200 nm | 2000 nm |
| None | 7 | 9 | 11 |
| Hydrogen fluoride (humid) | 12 | 12 | 15 |
| Ammonia (humid) | 9 | 10 | 13 |
| Sequentially exposed to both gases (humid) | 12 | 12 | 14 |

## Claims

1. A protective hood for protecting an individual from the effects of smoke and fire in a fire related emergency, comprising a high temperature resistant plastics material having a layer of titanium on at least a part of its outer surface.

2. A protective hood according to claim 1 in which the layer of titanium is from 100 to 1000 angstroms (10 to 100 nm) thick.

3. A protective hood according to claim 1 in which the layer of titanium comprises between 50 and 250 milligrams of titanium per square metre of plastics material.

4. A protective hood according to claim 3 in which the layer of titanium comprises between 100 and 150 milligrams of titanium per square metre of plastics material.

5. A protective hood according to claim 1 having a transmittance of electromagnetic radiation of between 10 and 40% at 620 nm.

6. A protective hood according to claim 5 having a transmittance of electromagnetic radiation of between 15 and 25% at 620 nm.

7. A protective hood according to claim 1 having about a 70% rejection of infra red radiation.

8. A protective hood according to any one of claims 1 to 7 in which at least a part of the layer of titanium is transparent to visible radiation.

9. A protective hood according to any one of the preceding claims in which the titanium has been applied by sputtering.

10. A protective hood according to any one of the preceding claims having a layer of fluoropolymer on at least a part of its inner surface.

11. A protective hood according to claim 10 in which the fluoropolymer is fluoroethylene polymer.

12. A protective hood according to claim 10 in which the fluoropolymer is perfluoroalkoxy polymer.

13. A protective hood according to any one of claims 10 to 12 in which the fluoropolymer in between 10 and 40 micrometres thick.

14. A protective hood according to any one of the preceding claims in which the high temperature resistant plastics material comprises a thermoset plastics material.

15. A protective hood according to claim 14 in which the thermoset plastics material comprises polyimide.

16. A protective hood according to any one of the preceding claims in which the plastics material is between 25 and 75 micrometres thick.

17. An emergency breathing apparatus comprising a protective hood according to any one of the preceding claims.

18. An emergency breathing apparatus according to claim 17 comprising a protective hood having filters to provide in use, breathable gas to an individual wearing the hood.

19. A protective garment comprising a high temperature resistant plastics material having a layer of titanium on at least a part of its outer surface.

20. A protective mask or shield comprising a high temperature resistant plastics material having a layer of titanium on at least part of its outer surface.

## Patentansprüche

1. Schutzhaube zum Schutz einer Person vor den Auswirkungen von Rauch und Feuer bei einem Notfall in Verbindung mit Feuer, die ein hochtemperatur-resistentes Plastmaterial umfaßt, das auf wenigstens einem Teil der Außenfläche eine Titanschicht hat.

2. Schutzhaube nach Anspruch 1, bei der die Titanschicht 100 bis 1000 Angström (10 bis 100 nm) stark ist.

3. Schutzhaube nach Anspruch 1, bei der die Titanschicht zwischen 50 und 250 mg Titan je m² Plastmaterial aufweist.

4. Schutzhaube nach Anspruch 3, bei der die Titanschicht zwischen 100 und 150 mg Titan je m² Plastmaterial aufweist.

5. Schutzhaube nach Anspruch 1, die einen Durchlässigkeitsgrad für elektromagnetische Strahlung von 10 bis 40% bei 620 nm hat.

6. Schutzhaube nach Anspruch 5, die einen Durchlässigkeitsgrad für elektromagnetische Strahlung von 15 bis 25% bei 620 nm hat.

7. Schutzhaube nach Anspruch 1, die eine Abweisung von Infrarotstrahlung von etwa 70% hat.

8. Schutzhaube nach einem der Ansprüche 1 bis 7, bei der wenigstens ein Teil der Titanschicht für sichtbare Strahlung durchlässig ist.

9. Schutzhaube nach einem der vorhergehenden Ansprüche, bei der das Titan durch Sputtern aufgebracht worden ist.

10. Schutzhaube nach einem der vorhergehenden Ansprüche, die zumindest auf einem Teil der Innenfläche eine Schicht eines Fluoropolymers hat.

11. Schutzhaube nach Anspruch 10, bei der das Fluoropolymer ein Fluorethylenpolymer ist.

12. Schutzhaube nach Anspruch 10, bei der das Fluoropolymer ein Perfluoralkoxypolymer ist.

13. Schutzhaube nach einem der Ansprüche 10 bis 12, bei der das Fluoropolymer 10 bis 40 µm stark ist.

14. Schutzhaube nach einem der vorhergehenden Ansprüche, bei der das hochtemperatur-resistente Plastmaterial ein hitzehärtbares Plastmaterial umfaßt.

15. Schutzhaube nach Anspruch 14, bei der das hitzehärtbare Plastmaterial ein Polyimid umfaßt.

16. Schutzhaube nach einem der vorhergehenden Ansprüche, bei der das Plastmaterial 25 bis 75 µm stark ist.

17. Notatmungsgerät, das eine Schutzhaube nach einem der vorhergehenden Ansprüche umfaßt.

18. Notatmungsgerät nach Anspruch 17, das eine Schutzhaube umfaßt, die Filter hat, um bei der Benutzung einer Person, welche die Haube tragt, ein für die Atmung geeignetes Gas bereitzustellen.

19. Schutzanzug, der ein hochtemperatur-resistentes Plastmaterial umfaßt, das auf wenigstens einem Teil der Außenfläche eine Titanschicht hat.

20. Schutzmaske oder Schutzschirm, die ein hochtemperatur-resistentes Plastmaterial umfassen, das auf wenigstens einem Teil der Außenfläche eine Titanschicht hat.

## Revendications

1. Capuchon protecteur pour protéger un individu des effets de la fumée et du feu en cas d'urgence par suite d'un incendie, comprenant un matériau plastique résistant aux températures élevées comportant une couche de titane sur au moins une partie de sa surface externe.

2. Capuchon protecteur selon la revendication 1, dans lequel la couche de titane à une épaisseur comprise entre 100 et 1000 Angström (10 à 100 nm).

3. Capuchon protecteur selon la revendication 1, dans lequel la couche de titane comprend de 50 à 250 milligrammes de titane par mètre carré de matériau plastique.

4. Capuchon protecteur selon la revendication 3, dans lequel la couche de titane comprend de 100 à 150 milligrammes de titane par mètre carré de matériau plastique.

5. Capuchon protecteur selon la revendication 1, ayant une transmission du rayonnement électromagnétique comprise entre 10 et 40% à 620 nm.

6. Capuchon protecteur selon la revendication 5, ayant une transmission du rayonnement électromagnétique comprise entre 15 et 25% à 620 nm.

7. Capuchon protecteur selon la revendication 1, ayant un rejet d'environ 70% du rayonnement infra-rouge.

8. Capuchon protecteur selon l'une quelconque des revendications 1 à 7, dans lequel au moins une partie de la couche de titane est transparente au rayonnement visible.

9. Capuchon protecteur selon l'une quelconque des revendications précédentes, dans lequel le titane a été appliqué par pulvérisation cathodique.

10. Capuchon protecteur selon l'une quelconque des revendications précédentes, comportant une couche de fluoropolymère sur au moins une partie de sa surface interne.

11. Capuchon protecteur selon la revendication 10, dans lequel le fluoropolymère est un polymère de fluoroéthylène.

12. Capuchon protecteur selon la revendication 10, dans lequel le fluoropolymère est un polymère de perfluoroalkoxy.

13. Capuchon protecteur selon l'une quelconque des revendications 10 à 12, dans lequel le fluoropolymère a une épaisseur comprise entre 10 et 40 micromètres.

14. Capuchon protecteur selon l'une quelconque des revendications précédentes, dans lequel le matériau plastique résistant aux températures élevées comprend un matériau plastique thermodurcissable.

15. Capuchon protecteur selon la revendication 14, dans lequel le matériau plastique thermodurcissable comprend du polyimide.

16. Capuchon protecteur selon l'une quelconque des revendications précédentes, dans lequel le matériau plastique a une épaisseur comprise entre 25 et 75 micromètres.

17. Appareil respiratoire d'urgence comprenant un capuchon protecteur selon l'une quelconque des revendications précédentes.

18. Appareil respiratoire d'urgence selon la revendication 17, comprenant un capuchon protecteur comportant des filtres pour fournir en service du gaz respirable à un individu portant le capuchon.

19. Vêtement de protection comprenant un matériau plastique résistant aux températures élevées comportant une couche de titane sur au moins une partie de sa surface externe.

20. Masque ou écran protecteur comprenant un matériau plastique résistant aux températures élevées comportant une couche de titane sur au moins une partie de sa surface externe.
